# EUROPEAN PATENT APPLICATION

(11) **EP 4 548 776 A1**
(43) Date of publication of application: **07.05.2025**
(21) Application number: 23831627.7
(22) Date of filing: 30.06.2023
(51) Int. Cl.: A23L 27/00, A23F 3/16, A23L 2/00, A23L 2/38, A23L 2/52, A23L 2/54, A23L 27/20, A61K 8/37, A61Q 11/00, A61Q 13/00, C12G 3/04

(54) **CARBONATION SENSATION IMPARTING OR ENHANCING AGENT**

(30) Priority: 30.06.2022 JP 2022105336
(71) Applicant: Takasago International Corporation, Tokyo 144-8721 (JP)
(72) Inventor: AKAI, Chie, Hiratsuka-shi, Kanagawa 254-0073 (JP); YAMADA, Taku, Hiratsuka-shi, Kanagawa 254-0073 (JP); KASHIWAGI, Takahiro, Hiratsuka-shi, Kanagawa 254-0073 (JP)
(74) Representative: Hoffmann Eitle
(86) International application number: PCT/JP2023/024388
(87) International publication number: WO 2024/005188

(57) **Abstract**

The present invention provides a carbonation sensation imparting or enhancing agent, etc. containing a diester compound represented by general formula (I) as an active ingredient. [In the formula, R is a methyl group, ethyl group, or isopropyl group, n is an integer of 1-8, and m is an integer of 0-6.]

## Description

### TECHNICAL FIELD

The present invention relates to a carbonation sensation imparting or enhancing agent, a carbonation sensation imparting or enhancing flavor composition, food, drink or oral care product to which fizziness is imparted or whose fizziness is enhanced, a method for producing the food, drink or the oral care product to which fizziness is imparted or whose fizziness is enhanced, a carbonation sensation imparting or enhancing method, a mouthfeel ameliorating agent, and a mouthfeel ameliorating method.

### BACKGROUND ART

A carbonated beverage is often drunk because of requiring unique stimulation to be felt in an oral cavity by a carbon dioxide gas in the beverage, and can give an exhilarating feeling or a refresh-feeling. Therefore, the carbonated beverage is a popular beverage in the market. In addition, with the diversification of the preference from consumers in recent years, the popularity of so-called strong carbonated beverages is also increasing.

There is a demand for consumers who desire stronger stimulation not only for carbonated beverages but also for food such as confectionery that give carbonated stimulation. Further, there is a need for a technique of imparting fizziness to foods or drinks without carbonic acid stimulation to which a carbonated beverage flavor or a fruit flavor is imparted by a flavor.

In order to enhance carbonic acid stimulation of carbonated beverages, a method for increasing the carbon dioxide gas pressure by increasing an amount of carbon dioxide gas added is generally used. However, when the carbon dioxide gas pressure is increased, there is a risk that a beverage is ejected during filling into a container or opening when drinking, and the carbon dioxide gas pressure that can be increased is also limited from the viewpoint of production equipment. Therefore, a method for enhancing fizziness without increasing the carbon dioxide gas pressure has been proposed in the related art.

For example, Patent Literature 1 proposes a carbonation sensation enhancing agent containing, as effective components, a capsicum extract, a pepper extract, a ginger extract, and a pungent component contained therein. Patent Literature 2 proposes a carbonation sensation enhancing method in which caffeine, quassin, and naringin, which are bitter components, are used as effective components. Patent Literature 3 proposes a carbonation sensation enhancing agent containing, as effective components, 3-hepten-2-one, 3-octen-2-one, 3-nonen-2-one, and 3-decen-2-one which have characteristic aromas. Patent Literature 4 proposes a carbonation sensation enhancing agent containing, as effective components, rotundone or the like which has a unique spicy and pepper-like aroma. Patent Literature 5 proposes a carbonic acid stimulation enhancing method in which methyl (3-oxo-2-pentylcyclopentyl)acetate, which is a compound generally added to food or drink as a flavor, is used as a raw material.

However, in the above-described technique, almost no carbonation sensation imparting effect is obtained, and the enhancing effect may not be exhibited in a wide addition concentration range.

In view of such a background, there is a demand for a new technique of imparting fizziness to products such as food, drink and oral care product or enhancing fizziness of the products.

### CITATION LIST

### PATENT LITERATURE

Patent Literature 1: JP2010-68749A
Patent Literature 2: JP2017-104046A
Patent Literature 3: JP2019-62867A
Patent Literature 4: JP2021-94024A
Patent Literature 5: WO2014/147886

### SUMMARY OF INVENTION

### TECHNICAL PROBLEM

An object of the present invention is to provide an agent for imparting fizziness to food, drink or oral care product, or enhancing fizziness thereof.

### SOLUTION TO PROBLEM

The present invention has been made in view of the above-described circumstances, and includes the following aspects.
[1] A carbonation sensation imparting or enhancing agent containing, as an effective component, a diester compound represented by the following general formula (I). 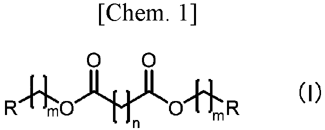 [In the formula, R represents a methyl group, an ethyl group, or an isopropyl group, n represents an integer of 1 to 8, and m represents an integer of 0 to 6.]
[2] The carbonation sensation imparting or enhancing agent according to the above-described [1], in which in the diester compound, R represents an ethyl group or an isopropyl group, n represents an integer of 2 to 8, and m represents an integer of 0 to 4.
[3] The carbonation sensation imparting or enhancing agent according to the above-described [1], in which the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.
[4] A carbonation sensation imparting or enhancing flavor composition containing the carbonation sensation imparting or enhancing agent according to any of above-described [1] to [3].
[5] Food, drink or oral care product to which fizziness is imparted or whose fizziness is enhanced, containing the carbonation sensation imparting or enhancing agent according to any one of the above-described [1] to [3] or the carbonation sensation imparting or enhancing flavor composition according to the above-described [4].
[6] The food, drink or the oral care product according to the above-described [5], in which a content of the diester compound is 0.01 ppm to 10000 ppm.
[7] A method for producing food, drink or oral care product to which fizziness is imparted or whose fizziness is enhanced, the method including a step of blending a diester compound in a content of 0.01 ppm to 10000 ppm in food, drink or oral care product, the diester compound being represented by the following general formula (I): 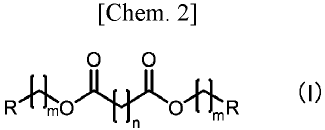 [In the formula, R represents a methyl group, an ethyl group, or an isopropyl group, n represents an integer of 1 to 8, and m represents an integer of 0 to 6.]
[8] The method for producing food, drink or oral care product to which fizziness is imparted or whose fizziness is enhanced according to the above-described [7], in which the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.
[9] A carbonation sensation imparting or enhancing method including blending a diester compound as an effective component into food, drink or oral care product, the diester compound being represented by the following general formula (I): 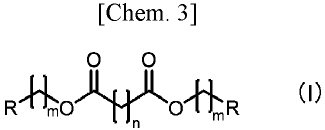 [In the formula, R represents a methyl group, an ethyl group, or an isopropyl group, n represents an integer of 1 to 8, and m represents an integer of 0 to 6.]
[10] The carbonation sensation imparting or enhancing method according to the above-described [9], in which the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.
[11] A mouthfeel ameliorating agent containing, as an effective component, a diester compound represented by the following general formula (I). 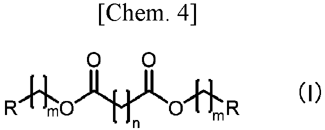 [In the formula, R represents a methyl group, an ethyl group, or an isopropyl group, n represents an integer of 1 to 8, and m represents an integer of 0 to 6.]
[12] The mouthfeel ameliorating agent according to the above-described [11], in which the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.
[13] A mouthfeel ameliorating method including blending a diester compound as an effective component into food, drink or oral care product, the diester compound being represented by the following general formula (I); 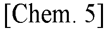 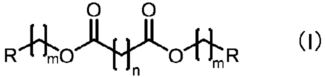 [In the formula, R represents a methyl group, an ethyl group, or an isopropyl group, n represents an integer of 1 to 8, and m represents an integer of 0 to 6.]
[14] The mouthfeel ameliorating method according to the above-described [13], in which the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.

### ADVANTAGEOUS EFFECTS OF INVENTION

According to a carbonation sensation imparting or enhancing agent or a carbonation sensation imparting or enhancing flavor composition in one aspect of the present invention, fizziness can be imparted to food, drink or oral care product, or fizziness of food, drink or oral care product can be enhanced.

### BRIEF DESCRIPTION OF DRAWINGS

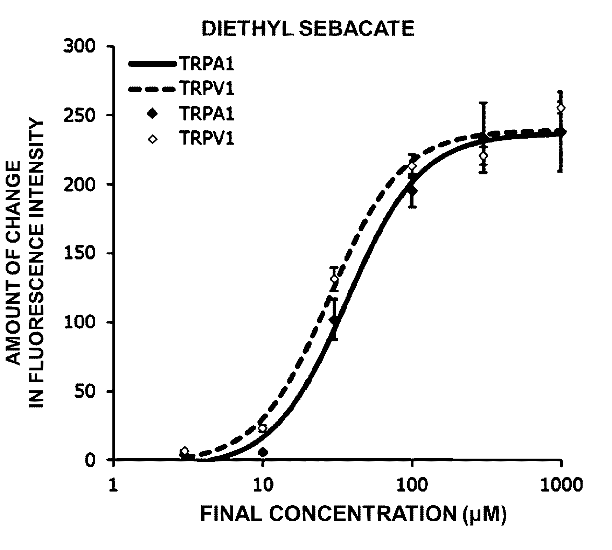

The FIGURE is a diagram showing that TRPV1 and TRPA1 respond to diethyl sebacate.

### DESCRIPTION OF EMBODIMENTS

The present invention will be described below. In the present description, the unit "ppm" means "ppm by mass". That is, in the present description, "ppm" means 0.001 mg/g. Further, the expression "to" indicating a numerical range is used in a meaning that numerical values described therebefore and thereafter are included as a lower limit value and an upper limit value.

In the present description, "carbonation sensation imparting or enhancing" means imparting fizziness or enhancing fizziness.

### <Carbonation sensation imparting or Enhancing Agent>

The carbonation sensation imparting or enhancing agent according to one aspect of the present invention can impart or enhance fizziness.

The carbonation sensation imparting or enhancing agent according to one aspect of the present invention can enhance, for example, fizziness caused by a carbon dioxide gas contained in food or drink, or fizziness caused by a carbon dioxide gas generated when food or drink exhibiting a stimulation specific to carbonic acid is put into an oral cavity. In addition, for example, fizziness can be imparted to food, drink or oral care product without original carbonic acid stimulation, which does not contain components that lead to fizziness (carbonic acid, sodium bicarbonate, etc.) and is flavored with a carbonated beverage flavor or fruit flavor, or the like by a flavor.

The carbonation sensation imparting or enhancing agent according to one aspect of the present invention contains, as an effective component, a diester compound represented by the following general formula (I).

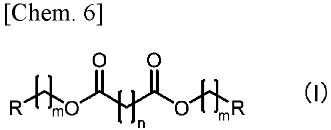

[In the formula, R represents a methyl group, an ethyl group, or an isopropyl group, n represents an integer of 1 to 8, and m represents an integer of 0 to 6.]

The above-described R is preferably an ethyl group or an isopropyl group, and more preferably an ethyl group.

The above-described n is preferably an integer of 2 to 8, and more preferably an integer of 4 to 8.

The above-described m is preferably an integer of 0 to 4, and more preferably an integer of 0 to 1.

In the present description, the diester compound represented by the formula (I) is preferably diethyl sebacate (n = 8, m = 0, R = ethyl group), diethyl succinate (n = 2, m = 0, R = ethyl group), diisobutyl adipate (n = 4, m = 1, R = isopropyl group), diethyl malonate (n = 1, m = 0, R = ethyl group), dibutyl sebacate (n = 8, m = 2, R = ethyl group), dimethyl sebacate (n = 8, m = 0, R = methyl group), or dipropyl adipate (n = 4, m = 1, R = ethyl group), more preferably diethyl sebacate, diethyl succinate, diisobutyl adipate, diethyl malonate, or dibutyl sebacate, and particularly preferably diethyl sebacate.

As the diester compound represented by the above-described formula (I), a diester compound obtained by chemical synthesis may be used, a diester compound obtained by extraction from a natural product may be used, or a combination thereof may be used. The above-described diester compounds may be used alone or in combination of two or more kinds thereof.

A preferable content of the above-described diester compound in the carbonation sensation imparting or enhancing agent according to one aspect of the present invention is appropriately determined depending on the purpose and the application, and is not particularly limited. The preferable content of the diester compound is preferably 0.01 mass% to 100 mass%, more preferably 0.02 mass% to 100 mass%, and still more preferably 0.05 mass% to 100 mass%.

The carbonation sensation imparting or enhancing agent according to one aspect of the present invention may contain various flavors in addition to the diester compound represented by the above-described formula (I). In the present specification, examples of various flavors include essential oils, natural flavors, and synthetic flavors described in, for example, "Official Gazette Known/Common Technical Book (Flavor or Fragrances) part II Food Flavors" (January 14, 2000, published by the Japan Patent Office). These flavors may be used alone or in combination of two or more kinds thereof.

### <Carbonation sensation imparting or Enhancing Method>

The present invention also provides a method for imparting fizziness to food, drink or oral care product or enhancing fizziness of food, drink or oral care product. The carbonation sensation imparting or enhancing method includes blending the diester compound as an effective component into food, drink or oral care product.

The carbonation sensation imparting or enhancing method means a method for imparting fizziness by blending the above-described diester compound, although there is no carbonic acid stimulation in a state where the above-described diester compound is not contained, or a method for enhancing fizziness by containing the above-described diester compound when fizziness is generated even in a state where the above-described diester compound is not contained.

In the present description, the expression "fizziness" means a unique stimulating sensation felt in a mouth or a throat when drinking a carbonated beverage containing a carbon dioxide gas. The stimulation generally brings about an exhilarating feeling or a refresh-feeling. Further, the expression "imparting fizziness" refers to newly adding fizziness, and the expression "enhancing fizziness" refers to increasing fizziness.

### <Carbonation sensation imparting or Enhancing Flavor Composition>

A carbonation sensation imparting or enhancing flavor composition according to one aspect of the present invention contains the carbonation sensation imparting or enhancing agent, and can be used in combination with the various flavors. A content of the diester compound represented by the formula (I) in the carbonation sensation imparting or enhancing flavor composition is appropriately determined depending on the purpose and the application, and is not particularly limited. The content of the diester compound is preferably 0.001 mass% to 99.5 mass%, more preferably 0.002 mass% to 99.5 mass%, and still more preferably 0.005 mass% to 99.5 mass%.

The carbonation sensation imparting or enhancing flavor composition according to one aspect of the present invention may further contain a solvent or a fixative. The solvent and the fixative are not particularly limited, and examples thereof include water, ethanol, isopropyl alcohol, ethylene glycol, propylene glycol, dipropylene glycol, glycerin, hexyl glycol, benzyl benzoate, triethyl citrate, diethyl phthalate, Hercolyn, medium chain triglyceride, and medium chain diglyceride. These solvents and the fixatives may be used alone or in combination of two or more kinds thereof.

The carbonation sensation imparting or enhancing flavor composition according to one aspect of the present invention may contain various additives. The additive is not particularly limited, and examples thereof include an antioxidant, a higher alcohol, an emulsifying agent, a surfactant, an ultraviolet absorber, a chelating reagent, a solubilizing agent, a stabilizing agent, a cooling agent, a preservative, an antibacterial agent, a bactericide, a dye, and a pH adjusting agent. These additives may be used alone or in combination of two or more kinds thereof.

### <Food or Drink or Oral Care Product>

One aspect of the present invention also relates to food, drink or oral care product to which fizziness is imparted or whose fizziness is enhanced, and a method for producing the food or drink or the oral care product. The expression "food, drink or oral care product to which fizziness is imparted or whose fizziness is enhanced" means food, drink or oral care product to which fizziness is imparted by containing the above-described diester compound, although there is no carbonic acid stimulation in a state where the above-described diester compound is not contained, or food, drink or oral care product whose fizziness is enhanced by containing the above-described diester compound when fizziness is generated even in a state where the above-described diester compound is not contained.

Those skilled in the art can appropriately determine, depending on properties of a target food or drink or a target oral care product or the required effect, the concentration of the carbonation sensation imparting or enhancing agent in the food or drink or the oral care product or the concentration of the carbonation sensation imparting or enhancing flavor composition therein. For example, the content of the diester compound represented by the formula (I), which is an effective component of the carbonation sensation imparting or enhancing agent according to one aspect of the present invention, in food, drink or oral care product is not particularly limited, and is preferably 0.01 ppm to 10000 ppm, more preferably 0.02 ppm to 5000 ppm, and still more preferably 0.05 ppm to 1000 ppm. The above content of the diester compound is not intended to be particularly limited, and more specifically, for example, the content of the above-described diester compound in a beverage is preferably 0.01 ppm to 100 ppm, more preferably 0.01 ppm to 50 ppm, still more preferably 0.02 ppm to 40 ppm, and particularly preferably 0.05 ppm to 20 ppm. The content of the above-described diester compound in a confection is preferably 0.05 ppm to 10000 ppm, more preferably 0.1 ppm to 5000 ppm, and still more preferably 0.25 ppm to 1000 ppm. The content of the above-described diester compound in an oral care product is preferably 0.05 ppm to 2000 ppm, more preferably 0.1 ppm to 1000 ppm, and still more preferably 0.25 ppm to 500 ppm.

### <Method for Producing Food or Drink or Oral Care Product>

The method for producing food, drink or oral care product to which fizziness is imparted or whose fizziness is enhanced includes a step of blending the diester compound into food, drink or oral care product. Note that "blending the diester compound into food, drink or oral care product" means that the diester compound is added from the outside of the food or drink or the oral care product.

The food or drink is not intended to be limited, and examples of the food or drink include beverages such as carbonated beverages, soft beverages, fruit juice beverages, milk beverages, lactic acid bacteria beverages, energy drinks, soy milks, and tea beverages; alcoholic beverages such as Chu-Hi, cocktails, sparkling wines, fruit wines, and herbal wines; desserts such as ice creams, ice milks, lacto ice creams, frozen desserts, yogurt, pudding, jelly, and daily desserts; confectionery such as caramels, candies, gummy candies, compressed tablet candies, crackers, biscuits, cookies, pies, chocolates, snacks, and chewing gums; and jams. Examples of the oral care product include tooth brushing powder, toothpaste, liquid toothpaste, mouthwash, and mouth spray.

In addition, the food or drink may be food or drink (hereinafter, also referred to as a "food or drink with carbonic acid stimulation") that exhibits a stimulation specific to carbonic acid. Examples of the food or drink with carbonic acid stimulation include carbonated beverages, frozen desserts, chewing gum, candies, gummy candies, compressed tablet candies, and jellies to which carbonic acid stimulation is imparted. In a non-beverage food, sodium bicarbonate and an acidulant (citric acid or the like) may coexist, and carbon dioxide gas may be generated when both components are dissolved in an oral cavity to impart carbonic acid stimulation, or when a high-pressure carbon dioxide gas-sealed candy in which high-pressure carbon dioxide gas is sealed in a hard candy may be used.

### <Mouthfeel Ameliorating Agent>

An agent containing the diester compound represented by the formula (I), which is an effective component of the carbonation sensation imparting or enhancing agent according to one aspect of the present invention, can also be used as a mouthfeel ameliorating agent. In the present description, the expression "mouthfeel" refers to a sensation different from taste, aroma, and appearance (visual sensation), and refers to the sensation felt in the oral cavity, including the teeth and tongue, mucosal sensation, or skin sensation when food, drink or oral care product is ingested. In the present description, the expression "mouthfeel" more specifically means a sensation such as "carbonic acid stimulation" or "foaming feeling". Here, the expression "carbonic acid stimulation" means, for example, a stimulating sensation including the stinging pain caused by carbonic acid and a burning feeling caused by an acid such as carbonic acid, and the expression "foaming feeling" more specifically means a feeling such as "carbonic acid stimulation". In addition, the expression "amelioration in mouthfeel" means ameliorating the above-described "mouthfeel", and specifically, means enhancing or imparting a sensation such as "heavy mouthfeel" or "minerality" of food, drink or oral care product. Here, the expressions "heavy mouthfeel" and "minerality" mean, for example, a salty taste and a slightly bitter taste felt from carbonic acid.

### <Mouthfeel Ameliorating Method>

The present invention also provides a mouthfeel ameliorating method. The mouthfeel ameliorating method includes blending the diester compound as an effective component into food, drink or oral care product.

Hereinafter, the present invention will be described in more detail with reference to Examples, but the present invention is not limited to these Examples.

### Examples

In the following Examples, the action and effect were verified using a value obtained by calculating an average score of scores for imparting or enhancing fizziness by sensory evaluation conducted by specialized panelists. The sensory evaluation was performed according to criteria shown in Table 1 below. The expression "1 point" in the evaluation is the evaluation in the case where no component is added at all (no addition).

### [Table 1]

**Table 1**

| (Fizziness enhancement evaluation criteria) |
|---|
| 5 points: fizziness is extremely enhanced |
| 4 points: fizziness is sufficiently enhanced |
| 3 points: fizziness is enhanced |
| 2 points: fizziness is slightly enhanced |
| 1 point: fizziness is not enhanced |
| |

| (Carbonation sensation imparting evaluation criteria) |
|---|
| 5 points: fizziness is extremely imparted |
| 4 points: fizziness is sufficiently imparted |
| 3 points: fizziness is imparted |
| 2 points: fizziness is slightly imparted |
| 1 point: almost no fizziness is felt |

### [Example 1] Carbonated Water (Raw Material: Water, Carbon Dioxide)

Diethyl sebacate (manufactured by Inoue Perfumery Mfg. Co., Ltd., the same applies to the following Examples) was separately added to "WILKINSON (registered trademark) TANSAN (manufactured by Asahi Soft Drinks Co., Ltd.)" so as to reach the concentrations shown in Table 2 below, and sensory evaluation was performed by seven specialized panelists to determine the extent to which fizziness was enhanced as compared with a control in which diethyl sebacate was not added. The average score of the scores is shown in Table 2.

### [Table 2]

**Table 2**

| Diethyl sebacate concentration | 0.1 ppm | 0.3 ppm | 1 ppm | 5 ppm | 10 ppm | 20 ppm | 50 ppm | 100 ppm |
|---|---|---|---|---|---|---|---|---|
| Fizziness enhancement evaluation result | 1.1 | 2.1 | 3.7 | 4.6 | 4.5 | 2.5 | 2.1 | 1.7 |

This experiment showed that fizziness of carbonated water can be enhanced by adding diethyl sebacate. In addition, "carbonic acid stimulation" is exemplified as a specific example of a sensation of "mouthfeel", and therefore, an effect of ameliorating the mouthfeel was also confirmed according to the above-described fizziness enhancement evaluation result.

### [Example 2] Carbonated Water (Raw Material: Water, Carbon Dioxide)

Diisobutyl adipate (manufactured by Inoue Perfumery Mfg. Co., Ltd., the same applies to the following Examples) was separately added to "WILKINSON (registered trademark) TANSAN (manufactured by Asahi Soft Drinks Co., Ltd.)" so as to reach the concentrations shown in Table 3 below, and sensory evaluation was performed by seven specialized panelists to determine the extent to which fizziness was enhanced as compared with a control in which diisobutyl adipate was not added. The average score of the scores is shown in Table 3.

### [Table 3]

**Table 3**

| Diisobutyl adipate concentration | 0.005 ppm | 0.01 ppm | 0.1 ppm | 0.5 ppm | 1 ppm | 5 ppm | 10 ppm |
|---|---|---|---|---|---|---|---|
| Fizziness enhancement evaluation result | 1.1 | 1.6 | 3.4 | 3.6 | 3.4 | 1.7 | 1.3 |

This experiment showed that fizziness of carbonated water can be enhanced by adding diisobutyl adipate. In addition, the effect of ameliorating the mouthfeel was also confirmed according to the above-described fizziness enhancement evaluation result.

### [Example 3] Carbonated Water (Raw Material: Water, Carbon Dioxide)

Dimethyl sebacate (manufactured by Tokyo Chemical Industry Co., Ltd.) was separately added to "WILKINSON (registered trademark) TANSAN (manufactured by Asahi Soft Drinks Co., Ltd.)" so as to reach the concentrations shown in Table 4 below, and sensory evaluation was performed by seven specialized panelists to determine the extent to which fizziness was enhanced as compared with a control in which dimethyl sebacate was not added. The average score of the scores is shown in Table 4.

### [Table 4]

**Table 4**

| Dimethyl sebacate concentration | 0.1 ppm | 0.5 ppm | 1 ppm | 5 ppm | 10 ppm | 20 ppm | 50 ppm | 100 ppm |
|---|---|---|---|---|---|---|---|---|
| Fizziness enhancement evaluation result | 1.1 | 1.6 | 2.0 | 2.4 | 3.1 | 2.6 | 1.4 | 1.3 |

This experiment showed that fizziness of carbonated water can be enhanced by adding dimethyl sebacate. In addition, the effect of ameliorating the mouthfeel was also confirmed according to the above-described fizziness enhancement evaluation result.

### [Example 4] Carbonated Water (Raw Material: Water, Carbon Dioxide)

Dipropyl adipate (manufactured by Tokyo Chemical Industry Co., Ltd.) was separately added to "WILKINSON (registered trademark) TANSAN (manufactured by Asahi Soft Drinks Co., Ltd.)" so as to reach the concentrations shown in Table 5 below, and sensory evaluation was performed by seven specialized panelists to determine the extent to which fizziness was enhanced as compared with a control in which dipropyl adipate was not added. The average score of the scores is shown in Table 5.

### [Table 5]

**Table 5**

| Dipropyl adipate concentration | 0.1 ppm | 0.5 ppm | 1 ppm | 5 ppm | 10 ppm | 20 ppm | 50 ppm | 100 ppm |
|---|---|---|---|---|---|---|---|---|
| Fizziness enhancement evaluation result | 1.1 | 1.4 | 1.9 | 2.6 | 3.0 | 1.7 | 1.4 | 1.1 |

This experiment showed that fizziness of carbonated water can be enhanced by adding dipropyl adipate. In addition, the effect of ameliorating the mouthfeel was also confirmed according to the above-described fizziness enhancement evaluation result.

### [Example 5] (Cider-flavored Carbonated Beverage)

Diethyl sebacate was separately added to "Mitsuya Cider (registered trademark) Zero Strong (manufactured by Asahi Soft Drinks Co., Ltd.)" so as to reach the concentrations shown in Table 6 below, and sensory evaluation was performed by seven specialized panelists to determine the extent to which fizziness was enhanced as compared with a control in which diethyl sebacate was not added. The average score of the scores is shown in Table 6.

### [Table 6]

**Table 6**

| Diethyl sebacate concentration | 0.1 ppm | 0.3 ppm | 1 ppm | 5 ppm | 10 ppm | 20 ppm | 50 ppm | 100 ppm |
|---|---|---|---|---|---|---|---|---|
| Fizziness enhancement evaluation result | 1.3 | 1.9 | 3.7 | 3.9 | 3.7 | 3.6 | 3.3 | 1.1 |

As a result, it was shown that fizziness of the cider-flavored carbonated beverage can be enhanced by adding diethyl sebacate. In addition, the effect of ameliorating the mouthfeel was also confirmed according to the above-described fizziness enhancement evaluation result.

### [Example 6] (Carbonated Grapefruit Flavored Chu-Hi)

Diethyl sebacate was separately added to "Hyoketsu (registered trademark) Sicilian lemon (manufactured by Kirin Brewery Co., Ltd.)" so as to reach the concentrations shown in Table 7 below, and sensory evaluation was performed by seven specialized panelists to determine the extent to which fizziness was enhanced as compared with a control in which diethyl sebacate was not added. The average score of the scores is shown in Table 7.

### [Table 7]

**Table 7**

| Diethyl sebacate concentration | 0.1 ppm | 0.3 ppm | 1 ppm | 5 ppm | 10 ppm | 20 ppm | 50 ppm | 100 ppm |
|---|---|---|---|---|---|---|---|---|
| Fizziness enhancement evaluation result | 1.1 | 1.7 | 4.0 | 4.0 | 3.9 | 3.4 | 2.6 | 1.3 |

As a result, it was shown that fizziness of the carbonated grapefruit flavored Chu-Hi can be enhanced by adding diethyl sebacate. In addition, the effect of ameliorating the mouthfeel was also confirmed according to the above-described fizziness enhancement evaluation result.

### [Example 7] (Beer-flavored Beverage)

Diethyl sebacate was separately added to "Asahi Dry Zero (registered trademark) (manufactured by Asahi Breweries, Ltd.)" so as to reach the concentrations shown in Table 8 below, and sensory evaluation was performed by seven specialized panelists to determine the extent to which fizziness was enhanced as compared with a control in which diethyl sebacate was not added. The average score of the scores is shown in Table 8.

### [Table 8]

**Table 8**

| Diethyl sebacate concentration | 0.1 ppm | 0.3 ppm | 1 ppm | 5 ppm | 10 ppm | 20 ppm | 50 ppm | 100 ppm |
|---|---|---|---|---|---|---|---|---|
| Fizziness enhancement evaluation result | 1.1 | 1.6 | 2.7 | 2.9 | 2.7 | 2.7 | 1.9 | 1.1 |

As a result, it was shown that fizziness of the carbonated beer-flavored beverage can be enhanced by adding diethyl sebacate. In addition, the effect of ameliorating the mouthfeel was also confirmed according to the above-described fizziness enhancement evaluation result.

### [Example 8] (Black Tea Beverage with Fruit Juice)

Diethyl sebacate was separately added to "Afternoon Tea Apple Tea Plus (registered trademark) (manufactured by Kirin Beverage Co., Ltd.)" so as to reach the concentrations shown in Table 9 below, and sensory evaluation was performed by seven specialized panelists to determine the extent to which fizziness was imparted as compared with a control in which diethyl sebacate was not added. The average score of the scores is shown in Table 9.

### [Table 9]

**Table 9**

| Diethyl sebacate concentration | 0.1 ppm | 0.3 ppm | 1 ppm | 5 ppm | 10 ppm | 20 ppm | 50 ppm | 100 ppm |
|---|---|---|---|---|---|---|---|---|
| Carbonation sensation imparting evaluation result | 1.1 | 1.3 | 1.9 | 2.7 | 2.9 | 1.7 | 1.7 | 1.1 |

As a result, it was shown that fizziness can be imparted to a black tea beverage with fruit juice, which contains no carbon dioxide, by adding diethyl sebacate. In addition, the effect of ameliorating the mouthfeel was also confirmed according to the above-described carbonation sensation imparting evaluation result.

### [Example 9] (Carbonation sensation enhancing Flavor Composition; Citrus-flavored Carbonated Water)

Citrus flavors were prepared according to the formulations shown in Table 10.

### [Table 10]

**Table 10**

| Citrus flavor formulation | | | | | | | | | | |
|---|---|---|---|---|---|---|---|---|---|---|
| | Weight ratio (%) | | | | | | | | | |
| Component | Comparative Example 1 | Example 9-1 | Example 9-2 | Example 9-3 | Example 9-4 | Example 9-5 | Example 9-6 | Example 9-7 | Example 9-8 | Example 9-9 |
| Citrusyol (*) | 0.10 | 0.10 | 0.10 | 0.10 | 0.10 | 0.10 | 0.10 | 0.10 | 0.10 | 0.10 |
| Diethyl sebacate | - | 0.02 | 0.06 | 0.10 | 0.20 | 1.00 | 2.00 | 4.00 | 10.00 | 20.00 |
| Ethanol | 99.90 | 99.88 | 99.84 | 99.80 | 99.70 | 98.90 | 97.90 | 95.90 | 89.90 | 79.90 |

| | | | | | | | | | | |
|---|---|---|---|---|---|---|---|---|---|---|
| *Registered trademark, manufactured by Takasago International Corporation; 3,7-dimethyl-2-methylene-6-octen-1-ol. | | | | | | | | | | |

Each of the above-described citrus flavors was added in an amount of 0.05 mass% to "WILKINSON (registered trademark) TANSAN (manufactured by Asahi Soft Drinks Co., Ltd.)", and sensory evaluation was performed by seven specialized panelists to evaluate whether the carbonation sensation enhancing effect was observed in Examples 9-1 to 9-9 compared with Comparative Example 1. The average score of the scores is shown in Table 11.

### [Table 11]

**Table 11**

| | Example 9-1 | Example 9-2 | Example 9-3 | Example 9-4 | Example 9-5 | Example 9-6 | Example 9-7 | Example 9-8 | Example 9-9 |
|---|---|---|---|---|---|---|---|---|---|
| Diethyl sebacate concentration of carbonated water (ppm) | 0.1 | 0.3 | 0.5 | 1 | 5 | 10 | 20 | 50 | 100 |
| Fizziness enhancement evaluation result | 1.3 | 1.7 | 3.7 | 4.5 | 3.6 | 2.5 | 2.5 | 2.1 | 1.1 |

As a result, it was shown that the flavor composition containing diethyl sebacate can enhance fizziness of carbonated water. In addition, the effect of ameliorating the mouthfeel was also confirmed according to the above-described fizziness enhancement evaluation result.

### [Example 10] (Carbonation sensation imparting Flavor Composition; Mineral Water) Lemonade flavors were prepared according to the formulations shown in Table 12.

### [Table 12]

**Table 12**

| | Weight ratio (%) | | | | | | | | |
|---|---|---|---|---|---|---|---|---|---|
| Component | Comparative Example 2 | Example 10-1 | Example 10-2 | Example 10-3 | Example 10-4 | Example 10-5 | Example 10-6 | Example 10-7 | Example 10-8 |
| Nonanal | 0002 | 0.002 | 0.002 | 0.002 | 0.002 | 0.002 | 0.002 | 0.002 | 0.002 |
| Citrusyol (*) | 0.010 | 0.010 | 0.010 | 0.010 | 0.010 | 0.010 | 0.010 | 0.010 | 0.010 |
| 1,8-cineole | 0.010 | 0.010 | 0.010 | 0.010 | 0.010 | 0.010 | 0.010 | 0.010 | 0.010 |
| Linalool | 0.150 | 0.150 | 0.150 | 0.150 | 0.150 | 0.150 | 0.150 | 0.150 | 0.150 |
| Alpha-terpineol | 0.200 | 0.200 | 0.200 | 0.200 | 0.200 | 0.200 | 0.200 | 0.200 | 0.200 |
| Citral | 0.500 | 0.500 | 0.500 | 0.500 | 0.500 | 0.500 | 0.500 | 0.500 | 0.500 |
| Diethyl sebacate | - | 0.010 | 0.050 | 0.100 | 0.500 | 1000 | 5.000 | 10.000 | 20.000 |
| Ethanol | 99.128 | 99.118 | 99.078 | 99.028 | 98.628 | 98.128 | 94.128 | 89.128 | 79.128 |

| | | | | | | | | | |
|---|---|---|---|---|---|---|---|---|---|
| *Registered trademark, manufactured by Takasago International Corporation; 3,7-dimethyl-2-methylene-6-octen-1-ol. | | | | | | | | | |

Each of the above-described lemonade flavors was added in an amount of 0.02% to "Suntory Natural Mineral Water (manufactured by Suntory Beverage & Food Limited.)", and sensory evaluation was performed by seven specialized panelists to evaluate whether the carbonation sensation imparting effect was observed in Examples 10-1 to 10-8 compared with Comparative Example 2. The average score of the scores is shown in Table 13.

### [Table 13]

**Table 13**

| | Comparative Example 2 | Example 10-1 | Example 10-2 | Example 10-3 | Example 10-4 | Example 10-5 | Example 10-6 | Example 10-7 | Example 10-8 |
|---|---|---|---|---|---|---|---|---|---|
| Diethyl sebacate concentration of mineral water (ppm) | 0 | 0.02 | 0.1 | 0.2 | 1 | 2 | 10 | 20 | 40 |
| Carbonation sensation imparting evaluation result | 1.0 | 1.1 | 1.9 | 2.1 | 3.7 | 3.9 | 3.6 | 1.9 | 1.3 |

As a result, it was shown that the flavor composition containing diethyl sebacate can impart fizziness to mineral water. In addition, the effect of ameliorating the mouthfeel was also confirmed according to the above-described carbonation sensation imparting evaluation result.

### [Example 11] (Confection)

Diethyl sebacate was separately added to a cola-flavored compressed tablet candy prepared according to the formulation shown in Table 14 using a pestle with a diameter of 7 mm, so as to reach a concentration shown in Table 15 below, and sensory evaluation was performed by six panelists to determine the extent to which fizziness was imparted as compared with a control in which diethyl sebacate was not added. The average score of the scores is shown in Table 15.

### [Table 14]

**Table 14**

| Formulation of cola-flavored compressed tablet candy | |
|---|---|
| Component | Part(s) by weight |
| Sorbitol | 93.50 |
| Sugar ester | 2.00 |
| Citric acid | 1.50 |
| Aspartame | 1.50 |
| Microparticulate diacid ketoacid | 0.50 |
| Cola flavor (*) | 1.00 |
| Diethyl sebacate | (Concentrations shown in Table 15) |

| | |
|---|---|
| * "Cola flavor M-24190" manufactured by Takasago International Corporation | |

### [Table 15]

**Table 15**

| | | | | | | | |
|---|---|---|---|---|---|---|---|
| Diethyl sebacate concentration | 0.1 ppm | 1 ppm | 10 ppm | 100 ppm | 1000 ppm | 10000 ppm | 20000 ppm |
| Carbonation sensation imparting evaluation result | 1.5 | 2.2 | 2.5 | 4.0 | 3.0 | 1.5 | 1.2 |

As a result, it was shown that fizziness can be imparted to compressed tablet candies containing no diethyl sebacate by adding diethyl sebacate. In addition, the effect of ameliorating the mouthfeel was also confirmed according to the above-described carbonation sensation imparting evaluation result.

### [Example 12] (Cider-flavored Frozen Dessert)

Diethyl sebacate was separately added to a cider-flavored frozen dessert prepared according to the formulation shown in Table 16 so as to reach a concentration shown in Table 17 below, and sensory evaluation was performed by seven panelists to determine the extent to which fizziness was imparted as compared with a control in which diethyl sebacate was not added. The average score of the scores is shown in Table 17.

### [Table 16]

**Table 16**

| Formulation of cider-flavored frozen dessert | |
|---|---|
| | Part(s) by weight |
| Fructose glucose liquid sugar | 10.00 |
| Fructose | 2.00 |
| Granulated sugar | 2.00 |
| Pectin | 0.30 |
| Citric acid | 0.25 |
| Salt | 0.05 |
| Water | 85.30 |
| Cider flavor (*) | 0.10 |
| Diethyl sebacate | (Concentrations shown in Table 17) |

| | |
|---|---|
| * Cider flavor manufactured by Takasago International Corporation | |

### [Table 17]

**Table 17**

| | | | | | | | | |
|---|---|---|---|---|---|---|---|---|
| Diethyl sebacate concentration | 0.1 ppm | 0.3 ppm | 1 ppm | 5 ppm | 10 ppm | 20 ppm | 50 ppm | 100 ppm |
| Carbonation sensation imparting evaluation result | 1.4 | 1.9 | 2.1 | 3.6 | 4.5 | 3.6 | 2.7 | 1.4 |

As a result, it was shown that fizziness can be imparted to the cider-flavored frozen dessert by adding diethyl sebacate. In addition, the effect of ameliorating the mouthfeel was also confirmed according to the above-described carbonation sensation imparting evaluation result.

### [Example 13] (Mouthwash)

Diethyl sebacate was separately added to grape-flavored mouthwash prepared according to the formulation shown in Table 18 so as to reach a concentration shown in Table 19 below, and sensory evaluation was performed by five panelists to determine the extent to which fizziness was imparted as compared with a control in which diethyl sebacate was not added. The average score of the scores is shown in Table 19.

### [Table 18]

**Table 18**

| Formulation of grape-flavored mouthwash | |
|---|---|
| Component | Part(s) by weight |
| Water | 84.47 |
| Glycerin | 10.00 |
| Ethanol | 5.00 |
| Hydrogenated castor oil | 0.40 |
| Sodium benzoate | 0.05 |
| Sodium saccharin | 0.01 |
| Grape flavor (*) | 0.07 |
| Diethyl sebacate | (Concentrations shown in Table 19) |

| | |
|---|---|
| * "Grape flavor G-83947" manufactured by Takasago International Corporation | |

### [Table 19]

**Table 19**

| | | | | | | | |
|---|---|---|---|---|---|---|---|
| Diethyl sebacate concentration | 0.1 ppm | 1 ppm | 5 ppm | 10 ppm | 50 ppm | 100 ppm | 500 ppm |
| Carbonation sensation imparting evaluation result | 1.8 | 2.2 | 2.8 | 3.4 | 4.0 | 3.8 | 3.0 |

As a result, it was shown that fizziness can be imparted to the mouthwash by adding diethyl sebacate. In addition, the effect of ameliorating the mouthfeel was also confirmed according to the above-described carbonation sensation imparting evaluation result.

### [Reference Example]

It was analyzed whether transient receptor potential ankyrin 1 (TRPA1) known as a carbonic acid receptor and transient receptor potential vanilloid 1 (TRPV1) responding to acids such as carbonic acid respond to diethyl sebacate which has been shown to enhance fizziness.

### <Preparation of Human TRPA1 Stably Expressing Cell Line and Human TRPV1 Stably Expressing Cell Line>

The full-length human TRPA1 gene (GenBank Accession Number: NM_007332) was amplified from plasmid pFN21AB7348 (manufactured by Kazusa DNA Research Institute), and the full-length human TRPV1 gene (GenBank Accession Number: NM_018727) was amplified from pFN21AE2418 (manufactured by Kazusa DNA Research Institute) using PCR. The obtained PCR product was subcloned into pcDNA5/FRT/TO (manufactured by ThermoFisher Scientific), and then introduced into Flp-In 293 cells (manufactured by Thermo Fisher Scientific) using a Flp-In T-REx system (manufactured by Thermo Fisher Scientific) to prepare a human TRPA1 stably expressing cell line and a human TRPV1 stably expressing cell line.

### <Evaluation of Human TRPA1 Activity and Human TRPV1 Activity>

The cultured human TRPA1 stably expressing cells and human TRPV1 stably expressing cells were seeded at a proportion of 50,000 cells/well to a collagen-coated 96-well microplate, and 1 µg/mL doxycycline (manufactured by Takara Bio Inc.) was added and cultured overnight at 37°C to induce expression of human TRPA1 and human TRPV1.

After replacing the culture medium with buffer solution, fluorescent calcium indicator Fluo 4-AM (manufactured by Dojindo Laboratories) was added and incubated at 37°C for 1 hour. After replacing the culture solution with buffer solution again, the microplate was set to a fluorescence microplate reader FlexStation 3 (manufactured by Molecular Devices, LLC.). Diethyl sebacate was added at various concentrations at an internal temperature of 27°C, and the fluorescence intensity at a wavelength of 525 nm when excited at a wavelength of 485 nm was measured. The changes in fluorescence intensity before and after the addition of the test substance were calculated, and the EC50 values were calculated to evaluate the TRPA1 and TRPV1 activity. The results are shown in the FIGURE. These results show that diethyl sebacate exhibits high TRPA1 activity and TRPV1 activity.

Although the present invention has been described in detail with reference to specific aspects, it will be apparent to those skilled in the art that various changes and modifications can be made without departing from the spirit and scope of the present invention. Note that, this application is based on a Japanese patent application (Japanese Patent Application No. 2022-105336) filed on June 30, 2022, which is incorporated by reference in its entirety. In addition, all references cited herein are incorporated in their entirety.

## Claims

1. A carbonation sensation imparting or enhancing agent comprising, as an effective component, a diester compound represented by the following general formula (I): 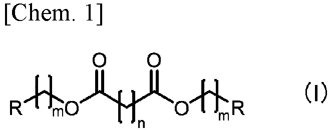 wherein R represents a methyl group, an ethyl group, or an isopropyl group, n represents an integer of 1 to 8, and m represents an integer of 0 to 6.

2. The carbonation sensation imparting or enhancing agent according to claim 1, wherein in the diester compound, R represents an ethyl group or an isopropyl group, n represents an integer of 2 to 8, and m represents an integer of 0 to 4.

3. The carbonation sensation imparting or enhancing agent according to claim 1, wherein the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.

4. A carbonation sensation imparting or enhancing flavor composition comprising the carbonation sensation imparting or enhancing agent according to any one of claims 1 to 3.

5. Food, drink or oral care product to which fizziness is imparted or whose fizziness is enhanced, comprising the carbonation sensation imparting or enhancing agent according to any one of claims 1 to 3 or the carbonation sensation imparting or enhancing flavor composition according to claim 4.

6. The food, drink or the oral care product according to claim 5, wherein a content of the diester compound is 0.01 ppm to 10000 ppm.

7. A method for producing food, drink or oral care product to which fizziness is imparted or whose fizziness is enhanced, the method comprising a step of blending a diester compound in a content of 0.01 ppm to 10000 ppm in food, drink or oral care product, the diester compound being represented by the following general formula (I): 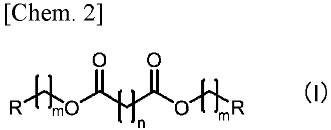 wherein R represents a methyl group, an ethyl group, or an isopropyl group, n represents an integer of 1 to 8, and m represents an integer of 0 to 6.

8. The method for producing food, drink or oral care product to which fizziness is imparted or whose fizziness is enhanced according to claim 7, wherein the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.

9. A carbonation sensation imparting or enhancing method comprising blending a diester compound as an effective component into food, drink or oral care product, the diester compound being represented by the following general formula (I): 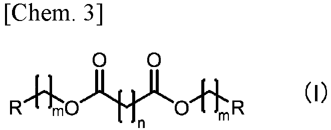 wherein R represents a methyl group, an ethyl group, or an isopropyl group, n represents an integer of 1 to 8, and m represents an integer of 0 to 6.

10. The carbonation sensation imparting or enhancing method according to claim 9, wherein the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.

11. A mouthfeel ameliorating agent comprising, as an effective component, a diester compound represented by the following general formula (I): 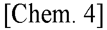 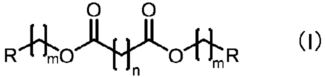 wherein R represents a methyl group, an ethyl group, or an isopropyl group, n represents an integer of 1 to 8, and m represents an integer of 0 to 6.

12. The mouthfeel ameliorating agent according to claim 11, wherein the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.

13. A mouthfeel ameliorating method comprising blending a diester compound as an effective component into food, drink or oral care product, the diester compound being represented by the following general formula (I): 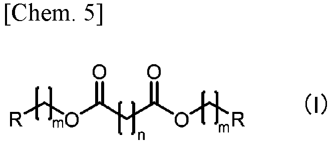 wherein R represents a methyl group, an ethyl group, or an isopropyl group, n represents an integer of 1 to 8, and m represents an integer of 0 to 6.

14. The mouthfeel ameliorating method according to claim 13, wherein the diester compound is one or more kinds of diester compounds selected from the group consisting of diethyl sebacate, diethyl succinate, diisobutyl adipate, diethyl malonate, dibutyl sebacate, dimethyl sebacate, and dipropyl adipate.
